# EUROPÄISCHE PATENTSCHRIFT

(11) **EP 0 495 341 B1**
(45) Veröffentlichungstag und Bekanntmachung des Hinweises auf die Patenterteilung: **05.07.1995**
(21) Anmeldenummer: 91810974.5
(22) Anmeldetag: 13.12.1991
(51) Int. Cl.: A61F 2/34, A61F 2/30

(54) **Implantat aus zwei Gleitpartnern**
Implant made of two conjugated sliding surfaces
Implant constitué de deux surfaces de frottement conjuguées

(30) Priorität: 18.01.1991 CH 145/91
(43) Veröffentlichungstag der Anmeldung: 22.07.1992
(73) Patentinhaber: SULZER Medizinaltechnik AG, 8404 Winterthur (CH)
(72) Erfinder: Koch, Rudolf, CH-8267 Berlingen (CH); Streicher, Robert Michael, CH-8405 Winterthur (CH)
(74) Vertreter: Trieblnig, Adolf

(56) Entgegenhaltungen:
- DE-A- 2 933 174
- GB-A- 1 189 325
- GB-A- 1 527 498

## Beschreibung

Die Erfindung betrifft ein Implantat aus zwei Gleitpartnern mit aufeinander gleitend bewegten Gleitflächen, wobei der eine Partner aus einem relativ weichen Material, zum Beispiel aus einem Kunststoff oder einem faserverstärkten Kunststoff, besteht, während der andere Partner aus einem relativ zum ersten harten Material, zum Beipiel aus einem Metall oder aus Biokeramik, hergestellt ist. Ein solches Implantat ist aus der GB-A-1527498 bekannt.

Bei Gelenkimplantaten, beispielsweise künstlichen Hüft- oder Kniegelenken, besteht der eine Gleitpartner sehr häufig aus einem relativ weichen Material, vorzugsweise einem Kunststoff oder einem faserverstärkten Kunststoff, um vor allem die stossdämpfenden und elastischen Eigenschaften des Kunststoffes zu nutzen, während der andere Gleitpartner aus einem relativ zum ersten Material harten Werkstoff, z.B. aus Biokeramik oder aus einem Metall, das gegebenenfalls mit einer Hartstoffauflage versehen sein kann, hergestellt ist. Ein bevorzugtes Material für den weichen Gleitpartner ist beispielsweise Polyäthylen, das unter Umständen mit Kohlefasern verstärkt sein kann.

Aus der CH-PS 449 173 ist eine metallische Pfannenschale für ein künstliches Hüftgelenk bekannt, in deren Gleitfläche Gleitkörper aus Kunststoff verteilt sind, die als elastisch federnde Elemente bei Belastungen zusammengedrückt werden und bei Entlastungen einen Spalt zwischen den Gleitflächen beider Gleitpartner öffnen.

In der Praxis hat sich nun gezeigt, dass die weichen Gleitpartner im Laufe der Zeit einen unzulässig hohen Abrieb erleiden, dessen Teilchen in dem das künstliche Gelenk umgebenden lebenden Gewebe zu unerwünschten Einschlüssen und damit zu Störungen und Schädigungen führen.

In der britischen Patentschrift GB 1527498 wird eine Hüftgelenkschale gezeigt, bei der viele harte Stützkörper vorzugsweise Einkristalle wie Rubine, als diskontinuierliche Gleitflächen in einer Halbkugelschale aus einem Elastomer eingebettet sind, damit eine künstliche Femurkugel auf ihnen gleiten kann. Diese Lösung hat den Nachteil, dass jeder der harten Stützkörper in engsten Toleranzen in Abhängigkeit zu den anderen in einer idealen Position für eine einzusetzende harte Kugel eingebettet sein muss. Da jedoch auch harte Kugeln Toleranzen unterworfen sind und da harte Oberflächen zu wenig elastisch sind, um sich aneinander anzupassen, entstehen immer wieder unerwünscht hohe hertzsche Pressungen, die auch an einer harten Kugel zu Verschleiss führen können.

Aufgabe der Erfindung ist es, Abrieb zu vermindern oder weitgehend zu vermeiden. Dieses Ziel wird mit den Kennzeichen vom unabhängigen Anspruch 1 erreicht.

In der Gleitfläche eines Gleitpartners aus weichem Material sind - beispielsweise ähnlich wie bei der vorstehend beschriebenen Metallschale die Gleitkörper - harte Stützkörper verteilt, die als abriebarme oder praktisch abriebfreie Gleitlagerflächen bei Belastungen des Gelenkes wirken. Um auf diesen Stützkörper eine möglichst grosse "Belastungsfläche" zu erreichen, sind deren Gleitflächen komplementär an die Gleitflächen des harten Gleitpartners angepasst, d.h. als Ausschnitte der negativen Form dieser Gleitflächen ausgebildet.

Um die "Einstellung" oder "Anpassung" der Stützkörper an die Gleitfläche oder die Gleitflächen des harten Partners zu erleichtern, sind die Auflageflächen, mit denen die Stützkörper in dem weichen Partner gelagert sind, und die Lagerflächen des weichen Gleitpartners für die Stützkörper bombiert. Bei derart bombierten Stützkörpern bzw. weichen Gleitpartnern ist es zusätzlich vorteilhaft, wenn die Mantelflächen der Stützkörper und die dazu komplementären Flächen des weichen Gleitpartners als Ausschnitte aus Kugelflächen ausgebildet sind, da dadurch eine gewisse Führung der Stützkörperbewegung in dem weichen Gleitpartner erfolgt.

Schliesslich ist es sinnvoll, wenn die Stützkörper zumindest der gleichen Materialklasse angehören wie der harte Gleitpartner, d.h. bei keramischem Gleitpartner aus Keramik und bei metallischem Gleitpartner aus Metall bestehen; selbstverständlich ist es auch möglich, die Stützkörper aus dem gleichen Material zu fertigen wie den harten Gleitpartner.

Im folgenden wird die Erfindung anhand eines Ausführungsbeispiels im Zusammenhang mit der Zeichnung näher erläutert.
- Fig. 1: zeigt eine Aufsicht auf den Pfannenhohlraum einer schematisch dargestellten Hüftgelenkspfanne;
- Fig. 2: ist ein Schnitt II-II von Fig. 1.

Die künstliche Hüftgelenkspfanne 1, die aus einem relativ weichen Material, beispielsweise aus Polyäthylen besteht, bildet den einen Gleitpartner einer Total-Hüftgelenksprothese; als zweiter Gleitpartner ist ein nicht dargestellter Gelenkkopf einer Femurkopfprothese vorgesehen, der aus einem harten Material, im vorliegenden Beispiel aus Biokeramik, hergestellt ist.

In über die Gleitfläche 5 verteilte Ausnehmungen 10 der Pfannenschale 2 sind daher Stützkörper 3 eingesetzt, die ebenfalls aus einem harten Material gefertigt sind. Die Stützkörper 3 gehören dabei mit Vorteil der gleichen Materialklasse wie der Gelenkkopf an oder - zumindest ihre Gleitfläche 4 - besteht aus dem gleichen Material wie diejenige des Gelenkkopfes. Wie erwähnt, ist im vorliegenden Beispiel ein Gelenkkopf aus biokeramischem Material angenommen; daher sind die Stützkörper 3 ebenfalls aus Biokeramik gefertigt.

Ihre Gleitfläche 4, die möglichst exakt in die Gleitfläche 5 der Pfannenschale 2 integriert ist, ist in ihrer Form, d.h. in ihrem Radius, an den Radius der Pfannenschale 2 angepasst, um zum einen eine möglichst grosse "Lagerfläche" auf einem Stützkörper 3 zu erhalten und zum anderen ein unbehindertes und abriebfreies Gleiten des Gelenkkopfes in der Schale 2 zu gewährleisten.

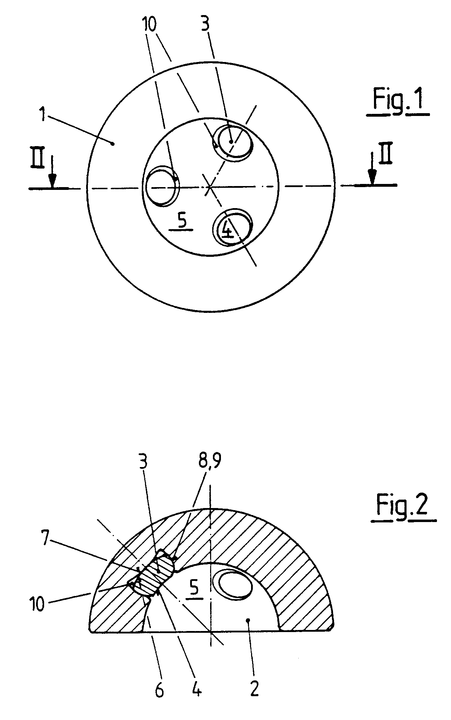

Wie Fig. 2 zeigt, sind sowohl die Lager- oder Auflageflächen 6, auf denen die Stützkörper 3 in der Pfanne 1 gelagert sind, als auch die Lager- oder Gegenflächen 7 der Pfanne 1 bombiert ausgeführt. Damit werden die Relativbewegungen der Stützkörper 3 in der Pfanne 1 erleichtert, so dass die Gleitflächen 4 des Stützkörpers 3 immer mit einer möglichst grossen Fläche an dem Gelenkkopf anliegen.

Um diese Relativbewegungen zu führen, sind sowohl die Mantelflächen 8 des Stützkörpers 3 als auch die dazu komplementären Flächen 9 der Pfanne 1 als Ausschnitte aus einer Kugel- bzw. einer Hohlkugelflächen ausgebildet.

## Patentansprüche

1. Implantat aus zwei Gleitpartnern mit aufeinander gleitend bewegten Gleitflächen, wobei der eine Partner (1) aus einem relativ weichen Material, zum Beispiel aus einem Kunststoff oder einem faserverstärkten Kunststoff, besteht, während der andere Partner aus einem relativ zum ersten harten Material, zum Beipiel aus einem Metall oder aus Biokeramik, hergestellt ist, wobei über die Gleitfläche (5) des weichen Gleitpartners (1) verteilt Stützkörper (3) mit Gleitflächen (4) aus einem harten Material eingelassen sind, die an die Form der Gleitflächen des harten Partners komplementär angepasst sind, dadurch gekennzeichet, dass die Stützkörper (3) in dem weichen Partner (1) beweglich gelagert sind, indem ihre auf dem weichen Partner (1) aufliegenden Auflageflächen (6) bombiert sind, während die Lagerflächen (7) des weichen Gleitpartners (1) für die Stützkörper (3) ebenfalls bombiert sind.

2. Implantat nach Anspruch 1, dadurch gekennzeichnet, dass die Mantelflächen (8) der Stützkörper (3) und die dazu komplementären Flächen (9) des weichen Gleitpartners (1) als Ausschnitte aus Kugelflächen ausgebildet sind.

3. Implantat nach Anspruch 1 oder 2, dadurch gekennzeichnet, dass die Stützkörper (3) der gleichen Materialklasse angehören wie der harte Gleitpartner.

4. Implantat nach einem der Ansprüche 1 bis 3, dadurch gekennzeichnet, dass die Stützkörper (3) aus dem gleichen Material wie der harte Gleitpartner bestehen.

## Claims

1. Implant comprising two members in sliding contact having sliding surfaces movable slidably in contact with each other, wherein one of the members (1) is of a relatively soft material, for instance of plastics or fibre-reinforced plastics, while the other is made of a material which is hard compared with the first mentioned material, for instance of a metal or bioceramics, wherein support elements (3) having sliding surfaces (4) of a hard material are set in distributed over the sliding surface (5) of the soft member (1), characterised in that the support elements (3) are fitted in the soft member (1) movably, in that their supporting surfaces (6) bearing on the soft member (1) are cambered, while the bearing surfaces (7) of the soft member (1) for the support elements (3) are also cambered.

2. Implant according to claim 1, characterised in that the circumferential surfaces (8) of the support elements (3) and the surfaces (9) of the soft member (1) complementary therewith are made as sections of spherical surfaces.

3. Implant according to claim 1 or 2, characterised in that the material of the support elements (3) is of the same class as that of the hard members.

4. Implant according to any one of claims 1 to 3, characterised in that the support elements (3) are made of the same material as the hard member.

## Revendications

1. Implant constitué de deux parties conjuguées de glissement avec surfaces de glissement se déplaçant l'une sur l'autre par glissement, une partie (1) étant réalisée dans une matière relativement souple, par exemple dans une matière plastique ou une matière plastique renforcée de fibres, tandis que l'autre partie est réalisée dans une matière dure par rapport à la première, par exemple dans un métal ou en biocéramique, des corps d'appui (3) avec surface de glissement (4) réalisée dans une matière dure étant encastrés, répartis sur la surface de glissement (5) de la partie (1) souple, lesquels corps d'appui sont adaptés de manière complémentaire à la forme des surfaces de glissement de la partie dure, caractérisé en ce que les corps d'appui (3) sont montés mobiles dans la partie souple (1), par le fait que leurs surfaces de support (6) reposant sur la partie souple (1) sont bombées, tandis que les surfaces d'appui (7) de la partie glissante souple (1) pour les corps d'appui (3) sont également bombées.

2. Implant selon la revendication 1, caractérisé en ce que les surfaces d'enveloppe (8) des corps d'appui (3) et les surfaces (9) complémentaires de la partie souple (1) sont réalisées sous la forme de découpes dans des surfaces sphériques.

3. Implant selon la revendication 1 ou 2, caractérisé en ce que les corps d'appui (3) font partie de la même classe de matière que la partie de glissement dure.

4. Implant selon l'une des revendications 1 à 3, caractérisé en ce que les corps d'appui (3) sont réalisés dans la même matière que la partie de glissement dure.
